Europäisches Patentamt

European Patent Office

Office européen des brevets

(19)

(11) Veröffentlichungsnummer: **0 503 425 A1**

# EUROPÄISCHE PATENTANMELDUNG

(12)

(21) Anmeldenummer: **92103551.5**

(22) Anmeldetag: **02.03.92**

(51) Int. Cl.⁵: **A61K 33/42**, A61K 9/50

(30) Priorität: **14.03.91 DE 4108239**

(43) Veröffentlichungstag der Anmeldung:
**16.09.92 Patentblatt 92/38**

(84) Benannte Vertragsstaaten:
**AT BE CH DE DK ES FR GB IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Lichtenberger, Rainer, Dr.**
**Heinrich-Fulda-Weg 19**
**W-6100 Darmstadt(DE)**
Erfinder: **Hanstein, Ullrich, Dr.**
**Schleifmühlenweg 4**
**W-6109 Mühltal(DE)**
Erfinder: **Hopfgartner,Johann**
**Hammerfeld 25**
**A-9800 Spittal(AT)**

(54) **Pellets enthaltend Natriummonofluorphosphat.**

(57) Natriummonofluorphosphat-Retardpellets, bestehend aus NaMFP-Pellets oder Neutralpellets mit aufgebrachtem Natriummonofluorphosphat, die mit einem magensaftbeständigen und/oder retardierenden Filmbildner überzogen sind.

EP 0 503 425 A1

Die Erfindung betrifft Pellets mit verzögerter Wirkstoffabgabe, die Natriummonofluorphosphat (NaMFP) enthalten.

NaMFP wird in der Medizin, ebenso wie NaF, zur prophylaktischen Behandlung und zur Bekämpfung von Osteoporose sowie verwandter osteologischer Krankheitsformen wie z.B. Osteomalazie und Morbus Paget eingesetzt (P.J. Meunier, Fortschr. Med. 108, 15-18 (1990)).

Die Wirkung des NaMFP beruht im wesentlichen auf der pharmakologischen Wirkung von Fluorid-Ionen auf den Knochen, wobei die Fluorid-Ionen bei der Resorption des Monofluorphosphats im Gastrointestinaltrakt durch Hydrolyse in der Darmmukosa entstehen (C.J. Spak, Brit. Med. Journal 289, 1686-1687 (1989)).

Fluorid-Ionen haben durch Stimulation der Osteoplasten nachweislich eine knochenmassenaufbauende Wirkung, sowohl im Spongiosabereich der Röhrenknochen, als auch im Vertebralbereich (U.S. 3287219; U.S. 4130630).

Die knochenaufbauende Wirkung wird durch die gleichzeitige Gabe von Calciumionen deutlich unterstutzt, wobei der Einsatz von Calciumcarbonat wegen der erhohten Calciumionen-Resorption im Vergleich zu anderen Calcium-Salzen bevorzugt wird. Beide Wirkstoffe, sowohl NaF als auch NaMFP, besitzen in den bislang bekannten Anwendungsformen erhebliche Nachteile.

So ist NaF als Wirkstoff wenig geeignet, da eine gleichzeitige Gabe von Ca-Ionen zu einer stark verminderten Fluoridionen-Resorption aufgrund der Bildung von schwerlöslichem $CaF_2$ führt. Weiterhin wirken freie Fluoridionen bei pH-Werten kleiner 2, wie sie im Magen vorherrschen, lokal toxisch und sind daher von hohen Nebenwirkungsraten begleitet.

NaMFP, welches diese Nachteile aufgrund des komplex gebundenen Fluors nicht aufweist, ist sehr hydrolyseempfindlich (J. Setnikar, Arzneim, Forsche, 38, 45-49 (1988)) und führt zudem bei gängigen Dosierungen, entsprechend 150-170 mg NaMFP, zu hohen Fluorid-Plasmaspiegeln, welche als Ursache für erhöhte Knochenbruchgefahr durch Versprödung oder auftretende Gelenkschmerzen verantwortlich gemacht werden können (P.D. Delmas et al., J. Bone Miner. Res. 5 Suppl. 1, 143-147 (1990)).

Bei allen bisher bekannten Darreichungsformen, in denen NaMFP in Kombination mit Ca-Salzen vorliegt, ist eine mehrmalige tägliche Einnahme erforderlich, um den Patienten mit einer ausreichenden Ca- bzw. Fluorid-Menge zu versorgen. Die Ca-Salze sind in keinem Beispiel in ausreichender Weise räumlich von dem hydrolyseempfindlichen NaMFP getrennt und tragen somit zu dessen vorzeitiger Zersetzung in erheblichem Maß bei, wobei gleichzeitig der wirksame Ca- bzw.

Fluorid-Gehalt durch Bildung von schwerlöslichem $CaF_2$ reduziert wird.

Der Erfindung lag die Aufgabe zugrunde, hydrolysestabile NaMFP enthaltende Darreichungsformen aufzufinden, die gleichzeitig Ca-Salze enthalten, jedoch eine gute räumliche Trennung beider Komponenten gewährleisten, zudem den Wirkstoff NaMFP mit zeitlicher Verzögerung abgeben, damit hohe Fluorid-Blutspiegelwerte und resultierende Nebenwirkungen vermeiden helfen (C.J. Pak, J. Clin. Endocrinol. Metabol., 278-286 (1989)) und darüber hinaus in einer sensorisch ansprechenden Form in einer täglichen Einmaldosis dem Patienten die erforderlichen Mengen an Calcium und Fluorid bereitstellen.

Es wurde gefunden, daß NaMFP enthaltende Pellets, die unter geeigneten Prozeßbedingungen mit einem magensaftbeständigen und/oder retardierendem polymeren Filmbildner überzogen sind, die erwünschte verzögerte Fluorid-Freisetzung zeigen, einen wirksamen langzeitigen Hydrolyseschutz bei Lagerung darstellen und eine gute räumliche Trennung von der zweiten essentiellen Komponente, dem Ca-Salz, gewährleisten. Die Pellets können in eine Calciumbrausetablette oder vorzugsweise in ein brausendes Granulat eingearbeitet und somit in einer sensorisch ansprechenden täglichen Einmaldosis, gegebenenfalls in Kombination mit geeigneten Geschmacksstoffen, dem Patienten in ausreichender Dosierung (min. 1,2 g $Ca^{2+}$ und 20 mg $F^-$ pro Tag) zur Verfügung gestellt werden. Diese Darreichungsformen zeichnen sich zudem durch eine leichte Applizierbarkeit im Vergleich zu Kapseln, Kautabletten oder Pastillen aus, die mit den genannten $Ca^-$ und Fluorid-Konzentrationen doch eine erhebliche Größe aufweisen, so daß eine Verteilung auf mehrmalige Gaben pro Tag erforderlich ist.

Gegenstand der Erfindung sind NaMFP-Retardpellets, bestehend aus Neutralpellets, mit aufgebrachtem NaMFP, dadurch gekennzeichnet, daß sie mit einem magensaftbeständigen und/oder retardierendem Filmbildner überzogen sind.

Als Neutralpellets dienen die bekannten handelsüblichen Formen (z.B. "Werner-Pellets" d. Fa. Werner), die in der Regel aus Zucker bestehen.

Als magensaftbeständige Filmbildner eignen sich an sich bekannte Polymere aus der Gruppe der Polymethylmethacrylate, der Celluloseacetatphthalate, Schellack oder besonders bevorzugt Hydroxypropylmethylcellulosephthalate, Ethylcellulose oder Polymethylmethacrylate.

Die erfindungsgemäßen NaMFP-Retardpellets können durch aufbauende Pelletisierung hergestellt werden, indem man den Wirkstoff in an sich bekannter Weise mit oder ohne Hilfsstoffe, unter Zuhilfenahme geeigneter Lösungsmittel, wie z.B. Alkoholen, Ketonen, Wasser oder Mischungen derselben, auf die vorgelegten Neutralpellets aufsprüht.

Als Hilfsstoffe eignen sich Stärke, insbesondere Maisstärke, Siliciumdioxid, bevorzugt hochdisperses $SiO_2$ und/oder Bindemittel, bevorzugt Polyvinylpyrrolidon (PVP), die die galenischen Eigenschaften verbessern.

Anschließend werden die Wirkstoffpellets nach an sich bekannter Methode in isolierte Pellets umgewandelt und mit einem magensaftbeständigen und/oder retardierendem Filmbildner überzogen, indem man diesen in Alkohol, vorzugsweise Ethanol oder Isopropanol oder Ketonen, vorzugsweise Aceton oder einer Alkohol/Keton-Mischung, vorzugsweise aus Isopropanol und Aceton bestehend, löst und, gegebenenfalls unter Zusatz von acetylierten Monogylceriden, aufsprüht.

Die Größe der erfindungsgemäßen Pellets kann bevorzugt zwischen 0,4 und 2,0 mm, besonders bevorzugt zwischen 0,7 und 1,3 mm variieren. Der NaMFP-Gehalt liegt vorzugsweise zwischen 10 und 90 %, besonders bevorzugt zwischen 30 und 50 %. Der Anteil des aufgebrachten Polymerfilms ist vom Durchmesser der Pellets abhängig und variiert vorzugsweise zwischen 5 und 50 %, besonders bevorzugt zwischen 7 und 35 %, insbesondere zwischen 9 und 15 % (alle Prozentangaben beziehen sich auf Gewichtsprozente).

Ferner ist Gegenstand der Erfindung ein Verfahren zur Herstellung pharmazeutischer Zubereitungen, insbesondere auf nicht-chemischem Wege, bei dem die NaMFP-Retardpellets zusammen mit mindestens einem Träger- oder Hilfsstoff und, falls erwünscht mit einem oder mehreren weiteren Wirkstoffen, bevorzugt mit Calciumcarbonat, aber auch mit anderen Calcium-Salzen, in eine geeignete Dosierungsform bringt.

Geeignete Träger- oder Hilfsstoffe sind Sorbit, Xylit, Saccharose, Maltit, Zitronen-, Äpfel-, Weinsäure, Saftpulver, wie z.B. Rote-Bete-Saftpulver, $\beta$-Carotin, Riboflavin, Tartrazin, Dioctylnatriumsulfosuccinat, Aspartam, Cyclamat, Acisulfam sowie andere an sich bekannte Zusätze, aber auch geeignete Geschmacks- und Aromastoffe können zusätzliche Bestandteile sein (W. Stammberger, Acta Pharm. Technol. Suppl. 1, 7-16 (1976)).

Die NaMFP-Retardpellets können zur Herstellung eines Arzneimittels verwendet werden und bei der Therapie von Erkrankungen des menschlichen oder tierischen Körpers verabreicht werden, insbesondere bei der Therapie und/oder Prophylaxe von Knochenerkrankungen, wie z.B. Osteoporose, Osteomalazie, Morbus Paget oder anderen osteologischen Krankheitsformen.

Bei allen vor- und nachstehenden Prozentangaben handelt es sich um Gewichtsprozente, während alle Temperaturen in ° C angegeben sind.

## Beispiele

### Beispiel 1

112,60 kg käufliche Neutralpellets herkömmlicher Art werden in einem Dragierkessel mit 8,18 kg Polyvinylpyrrolidon, gelöst in einer Mischung aus 42,3 l Isopropanol, 14,1 l Aceton und 9,8 l hochreinem Wasser (aqua purificata), besprüht und anschließend portionsweise mit einem Gemisch, bestehend aus 166,74 kg NaMFP, 4,16 kg hochdispersem $SiO_2$ und 8,32 kg Maisstärke, belegt. Die erhaltenen Wirkstoffpellets werden nach Trocknung bei 30-35 ° C auf herkömmliche Weise zu isolierten Wirkstoffpellets verarbeitet und einem Retardiervorgang unterworfen (s. Bsp. 2). Man erhält magensaftresistente NaMFP-Pellets mit Retardeigenschaft.

### Beispiel 2

Zur Retardierung werden 300 kg der isolierten Wirkstoffpellets aus Beispiel 1 in einem Dragierkessel vorgelegt und mit einer Losung bestehend aus 507,7 l Aceton, 640,5 l Isopropanol, 60,76 kg Hydroxypropylmethylcellulosephthalat, und etwa 6 kg acetyliertem Monoglycerid besprüht. Es wird bei einer konstanten Temperatur von 25° gearbeitet, wobei bedarfsweise etwas Talk zugefügt werden kann. Nach Retardierung wird 1 Std. im Warmluftstrom bei 35° getrocknet.

### Beispiel 3

Man verfährt analog Beispiel 2, nur setzt man zur Beschichtung der isolierten Wirkstoffpellets anstelle von Hydroxypropylmethylcellulosephthalat Polymethylmethacrylate ein.

### Beispiel 4

Man verfahrt analog Beispiel 2, nur setzt man zur Beschichtung der isolierten Wirkstoffpellets anstelle von Hydroxypropylmethylcellulosephthalat Celluloseacetatphthalat ein.

### Beispiel 5

Zar Herstellung eines NaMFP-Retardpellets enthaltenden brausenden Calcium-Granulats werden 3 kg $CaCO_3$ (socal E2, DAB), 2.3 kg Sorbit, 5 kg Citronensäure, 20 g Rote-Bete-Saftpulver, 50 g $\beta$-Carotin, 1 g Dioctylnatriumsulfosuccinat, je 25 g Pfirsich- und Aprikosenaroma, 50 g Aspartam sowie 360 g NaMFP-Retardpellets (s. Bsp. 2) unter Verwendung von 300 ml hochreinem Wasser und 700 ml Ethanol in üblicher Weise in einem Schnekkenmischer gemischt, auf einem Hordenblech bei 40° 36 Std. getrocknet, über ein Sieb zur gewünschten Große gebrochen und, nach der Qualitätskontrolle, portioniert und verpackt.

Beispiel 6

Analog Beispiel 4 werden die Bestandteile der Wirkstoff-Kombination im Wirbelschichtverfahren zu einem Granulat verarbeitet, indem eine vorgelegte Mischung $CaCO_3$/Citronensäure im Trockenluftstrom mit einer Sorbitlösung besprüht, die trockene Feststoffmischung über ein Sieb granuliert und anschließend mit den übrigen Zusatzstoffen im Schneckenmischer vermischt wird.

Beispiel 7

In eine Wirbelschichtanlage Glatt 120 oder Wurster 120 werden 100,0 kg isolierte Natriummonofluorphosphat Pellets vorgelegt und durch Besprühung mit Hydroxypropylmethylcellulosephthalat, gelöst in einer Aceton-Isopropanolmsichung, besprüht. Die Besprühung der Pellets erfolgt über eine Einstrahl- oder Mehrstrahldüse. Zu- und Abluftmengen werden so eingestellt, daß die Pellets in einem optimalen Schwebezustand verweilen. Während der Besprühung ist eine Korrektur der Zu- und Abluft erforderlich. Als Zusatz- oder Hilfsstoffe werden Myvacet und Talk verwendet. Nach Beendigung der Besprühung werden die Pellets nachgetrocknet und anschließend ausgesiebt.

**Patentansprüche**

1. Natriummonofluorphosphat (NaMFP)-Retardpellets, bestehend aus Neutralpellets mit aufgebrachtem NaMFP, die mit einem magensaftbeständigen und/oder retardierendem Filmbildner überzogen sind.

2. Verfahren zur Herstellung von NaMFP-Retardpellets nach Anspruch 1 durch aufbauende Pelletisierung, dadurch gekennzeichnet, daß man NaMFP auf Neutralpellets aufbringt und anschließend mit einem magensaftbeständigen und/oder retardierendem Filmbildner überzieht.

3. Verfahren zur Herstellung pharmazeutischer Zubereitungen, dadurch gekennzeichnet, daß man NaMFP-Retardpellets nach Anspruch 1 zusammen mit mindestens einem Träger- oder Hilfsstoff in eine geeignete Dosierungsform bringt.

4. Verwendung von NaMFP-Retardpellets nach Anspruch 1 zur Herstellung eines Arzneimittels zur prophylaktischen Behandlung oder zur Bekämpfung von knochenerkrankungen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 127 984 (KLINGE PHARMA GMBH) 3. Februar 1983<br>* Ansprüche *<br>* Seite 5, Zeile 22 - Seite 6, Zeile 16 *<br>* Seite 7 - Seite 8; Beispiel *<br>--- | 1-4 | A61K33/42<br>A61K9/50 |
| A | GB-A-2 195 246 (COLGATE-PALMOLIVE COMPANY) 7. April 1988<br>* das ganze Dokument *<br>--- | 1-4 | |
| A | GB-A-2 195 245 (COLGATE-PALMOLIVE COMPANY) 7. April 1988<br>* das ganze Dokument *<br>--- | 1-4 | |
| A | EP-A-0 383 967 (DOJIN IYAKU-KAKO CO.,LTD) 29. August 1990<br>* Seite 5 - Seite 6; Beispiel 1 *<br>----- | 1-4 | |
| | | | **RECHERCHIERTE SACHGEBIETE (Int. Cl.5)**<br><br>A61K |

Der vorliegende Recherchenbericht wurde für alle Patentansprüche erstellt

| Recherchenort | Abschlußdatum der Recherche | Prüfer |
|---|---|---|
| DEN HAAG | 15 JUNI 1992 | BOULOIS D. |

**KATEGORIE DER GENANNTEN DOKUMENTE**

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
......................................................................
& : Mitglied der gleichen Patentfamilie, übereinstimmendes
    Dokument

EPO FORM 1503 03.82 (P0403)